# EUROPÄISCHE PATENTANMELDUNG

(11) **EP 3 811 997 A1**
(43) Veröffentlichungstag der Anmeldung: **28.04.2021**
(21) Anmeldenummer: 19204609.2
(22) Anmeldetag: 22.10.2019
(51) Int. Cl.: A61M 25/10, A61M 25/01

(54) **BALLONDILATATION MIT GEPULSTEN MAGNETFELDERN**

(71) Anmelder: BIOTRONIK AG, 8180 Bülach (CH)
(72) Erfinder: Fargahi, Amir, 8180 Bülach (CH)
(74) Vertreter: Randoll, Sören

(57) **Zusammenfassung**

Die Anmeldung betrifft ein Ballonkathetersystem (1), mit: einem Ballonkatheter (2) aufweisend einen Katheterschaft (5) und einen am distalen Ende des Katheterschafts angeordneten Ballon (6), der eine dehnbaren Ballonwandung (60) aufweist, die einen Balloninnenraum (6a) des Ballons (6) umgibt, und einem fluiden Inflationsmedium (7), das zum Inflatieren des Ballons (6) über ein Lumen (5a) des Katheterschafts (5) in den Balloninnenraum (6a) einfüllbar ist. Erfindungsgemäß ist vorgesehen, dass das fluide Inflationsmedium (7) ein Ferrofluid ist, wobei das Ballonkathetersystem (1) weiterhin eine Spule (3) zum Erzeugen eines magnetischen Feldes aufweist und einen mit der Spule (3) verbundenen Pulsgenerator (4), der dazu konfiguriert ist, in der Spule (3) aufeinander folgende Strompulse zu erzeugen, so dass die Spule (3) ein zeitlich veränderliches Magnetfeld erzeugt, um eine Viskosität des Inflationsmediums (7) aufgrund des magnetorheologischen Effekts alternierend zu erhöhen und zu senken, wenn sich das Inflationsmedium (7) im Balloninnenraum (6a) befindet.

## Beschreibung

Die Erfindung betrifft ein Ballonkathetersystem sowie ein Verfahren zum Aufweiten oder bzw. Rekanalisieren einer Stenose in einem Gefäß eine Patienten.

Hierbei wird der Ballon des Ballonkatheters im Blutgefäß am Eingang der zu behandelnden Stenose platziert, so dass die Stenose durch Inflatieren des Ballons mit einem fluiden Inflationsmedium, das in den Balloninnenraum eingeleitet wird, aufgeweitet bzw. rekanalisiert werden kann. Beim Inflatieren dehnt sich der Ballon insbesondere in der radialen Richtung aus (d.h. senkrecht zur axialen Richtung des Ballonkatheters), wobei der Durchmesser des Ballons in der radialen Richtung entsprechend zunimmt. Weiterhin kann bei einem solchen Eingriff ein Stent eingesetzt werden, der anfänglich auf dem Ballon angeordnet ist und beim Inflatieren des Ballons aufgeweitet und in die eröffnete Stenose eingesetzt wird. Rekanalisieren bedeutet insbesondere, dass ein Verschluss des Gefäßes im Bereich der Stenose wieder durchgängig gemacht wird.

Zum Aufbrechen bzw. Rekanalisieren von Stenosen wird hierbei oftmals ein vergleichsweise hoher Druck im Ballon verwendet, wodurch das Risiko einer Ruptur des verengten Gefäßes steigt. Weiterhin sind Verfahren bzw. Katheter bekannt, die zum Wiedereröffnen einer Stenose Ultraschall verwenden.

Hiervon ausgehend liegt der vorliegenden Erfindung die Aufgabe zugrunde, einen Ballonkatheter bereitzustellen, der es gestattet die Dilation sowie insbesondere eine Stent-Freigabe mit einem niedrigeren Druck durchzuführen.

Diese Aufgabe wird durch einen Ballonkatheter mit den Merkmalen des Anspruchs 1 gelöst. Vorteilhafte Ausgestaltungen des Ballonkatheters sind in den entsprechenden Unteransprüchen angegeben und werden nachfolgend beschrieben.

Demgemäß wird ein Ballonkatheter zum Aufweiten bzw. Rekanalisieren einer Stenose vorgeschlagen, mit:
- einem Ballonkatheter, der einen Katheterschaft und einen Ballon aufweist, wobei der Ballon an einem distalen Ende des Katheterschafts festgelegt ist, und wobei der Ballon eine dehnbaren Ballonwandung aufweist, die einen Balloninnenraum des Ballons umgibt, und
- einem fluiden Inflationsmedium, das zum Inflatieren des Ballons über ein Lumen des Katheterschafts in den Balloninnenraum einfüllbar ist.

Erfindungsgemäß ist vorgesehen, dass das fluide Inflationsmedium ein Ferrofluid ist, wobei das Ballonkathetersystem weiterhin eine Spule zum Erzeugen eines magnetischen Feldes aufweist und einen mit der Spule elektrisch leitend verbundenen Pulsgenerator, der dazu konfiguriert ist, in der Spule aufeinander folgende Strompulse zu erzeugen, so dass die Spule ein zeitlich veränderliches Magnetfeld erzeugt, um eine Viskosität des Inflationsmediums aufgrund des magnetorheologischen Effekts abwechselnd zu erhöhen und zu senken, wenn sich das Inflationsmedium im Balloninnenraum befindet.

Durch das Magnetfeld richten sich die magnetischen Partikel des Ferrofluids aus, wodurch sich die Viskosität des Inflationsmediums bzw. Ferrofluids schlagartig stark erhöht. Bei einem ausgeschalteten Magnetfeld sinkt die Viskosität wieder ab.

Aufgrund der periodisch schwankenden Viskosität des Inflationsmediums im Balloninnenraum sind mit dem inflatierten Ballon Kraftstöße auf die Stenose mit einer hohen Frequenz möglich, die das Aufbrechen der Stenose durch Ermüdung des stenosen Materials erleichtern. Aufgrund der Möglichkeit, aufeinanderfolgende Kraftstöße auf die Stenose auszuüben, kann grundsätzlich ein geringerer maximaler Druck im inflatierten Ballon verwendet werden. Somit wird eine optimale Rekanalisation mit insgesamt niedrigerem Ballondruck und damit reduziertem Risiko einer Ruptur des Gefäßes erzeugt.

Sofern im Rahmen der vorliegenden Erfindung von einer proximalen Komponente oder einem proximalen Bereich und einer entsprechenden distalen Komponente bzw. einem distalen Bereich des Ballonkatheters die Rede ist, so bedeutet das, dass die distale Komponente bzw. der distale Bereich entlang des Verlaufs des Katheterschafts weiter von einem Bediener des Ballonkatheters entfernt ist, als die proximale Komponente bzw. der proximale Bereich. Bei dem Bereich kann es sich z.B. um einen Endabschnitt oder ein Ende einer Komponente des Ballonkatheters handeln.

Gemäß einer Ausführungsform der Erfindung ist vorgesehen, dass das Ferrofluid, ferromagnetische Partikel (z.B. in Form von Eisenpartikeln) sowie eine Trägerflüssigkeit aufweist, wobei die besagten Partikel in der Trägerflüssigkeit suspendiert sind.

In einer Ausführungsform ist das Ferrofluid eine Wasserdispersion von Eisenoxidpartikeln ist, die vorzugsweise aus Magnetit (Fe₃O₄) und Maghemit (γ-Fe₂O₃) bestehen. In einer anderen Ausführungsform ist das Ferrofluid eine Wasserdispersion von Cobalteisenoxidpartikern, die vorzugsweise aus Cobaltferrit-Partikeln (CoFe₂O₄) bestehen. In einer anderen Ausführungsform ist das Ferrofluid eine Wasserdispersion von Nickeleisenoxidpartikern, die vorzugsweise aus Nickelferrit-Partikeln (NiFe₂O₄) bestehen. In einer weiteren Ausführungsform kann das Ferrofluid eine Wasserdispersion mit einer Mischung der zuvor genannten Oxide sein. Dabei kann eine Mischung aus nur zwei der drei Oxide vorliegen oder auch ein Mischung Aus allen drei Oxiden.

In einer weiteren Ausführungsform liegen die Eisenoxidpartikel im Ferrofluid mit Aminosilanen beschichtet vor.

Weiterhin ist gemäß einer Ausführungsform der Erfindung vorgesehen, dass die ferromagnetischen Partikel (z.B. Eisenpartikel) im Inflationsmedium mit einem Massenanteil im Bereich von 10 Gew.-% bis 30 Gew.-% vorliegen.

Weiterhin ist gemäß einer Ausführungsform der Erfindung vorgesehen, dass die ferromagnetischen Partikel (z.B. Eisenpartikel) im Inflationsmedium mit einerVolumenkonzentration in der Trägerflüssigkeit von 2 Vol.-% bis 15 Vol.-% vorliegen.

Weiterhin ist gemäß einer Ausführungsform der Erfindung vorgesehen, dass die Dichte des Inflationsmediums bzw. Ferrofluids im Bereich von 1,0 g·cm⁻³ bis 4 g·cm⁻³, vorzugsweise 1,2 g·cm⁻³ bis 2,5 g·cm⁻³ liegt.

Weiterhin ist gemäß einer Ausführungsform der Erfindung vorgesehen, dass die ferromagnetischen Partikel einen Durchmesser im Bereich von 5 nm bis 25nm aufweisen, bevorzugt 8 nm bis 16 nm, wobei vorzugsweise der Durchmesser durchschnittlich etwa 10 nm beträgt. Die ferromagnetischen Partikel (z.B. Eisenpartikel) können kugelförmig ausgebildet sein. Ferner kann es bevorzugt sein, dass die Partikel in Durchmesser kleiner 10 nm sind.

In einer Ausführungsform hat das Inflationsmedium einen Magnetisierungskoeffizient von mindestens 3 l/g. In einer weiteren Ausführungsform hat das Inflationsmedium eine Magnetisierung I von mindestens 200 A/m. Das Inflationsmedium kann auch beide zuvor genannten Merkmale gleichzeitig erfüllen.

Inflationsmedien mit den zuvor genannten Eigenschaften, insbesondere, wenn sie aus Eisenpartikeln bestehen, haben den Vorteil, dass sie eine geringe Toxizität aufweisen und für menschliche Zellen verträglich sind. Ferner kann so eine kostengünstige Synthese des Inflationsmediums sichergestellt werden.

Weiterhin ist gemäß einer Ausführungsform der Erfindung vorgesehen, dass der Pulsgenerator dazu konfiguriert ist, Strompulse in der Spule zu erzeugen, die eine Wiederholrate im Bereich von 100Hz (50-150 Hz) aufweisen. Dabei ist es ferner bevorzugt, wenn die Pulse mit einer Magnetfeldstärke von 15 bis 20 mTesla vorgenommen werden, ferner rechteckigen Form aufweisend.

Weiterhin ist gemäß einer Ausführungsform der Erfindung vorgesehen, dass die Spule mobil ist. In einer Ausführungsform sind sowohl Spule als auch Pulsgenerator außerhalb des Körpers des Patienten angeordnet, also im proximalen Bereich des Ballonkatheters. In einer weiteren Ausführungsform ist die Spule auf den Innenschaft im Bereich des Ballon angeordnet und über eine Leitung mit dem Pulsgenerator verbunden, während der Pulsgenerator außerhalb des Patienten verbleibt. In einer weiteren Ausführungsform ist die Spule unabhängig vom Ballonkatheter angeordnet und wirkt über Fernübertragung auf das Inflationsmedium ein. In dieser Ausführungsform kann die Spule beispielsweise in einem Röntgengerät angeordnet sein. In dieser Ausführungsform würde sich die Spule in einem Abstand von 10 - 50 cm entfernt vom Ballonkatheter befinden.

Weiterhin ist gemäß einer Ausführungsform der Erfindung vorgesehen, dass das Ballonkathetersystem einen Stent aufweist, der auf den Ballon gecrimpt ist. Der Stent dient nach dem Aufweiten bzw. Rekanalisieren der Stenose dazu, das Gefäß im Bereich der aufgeweiteten Stenose zu stützen.

Ein weiterer Aspekt der Erfindung betrifft ein Verfahren zum Aufweiten und/oder Rekanalisieren einer Stenose mittels eines Ballonkathetersystems nach einem der vorhergehenden Ansprüche, aufweisend die Schritte:
- Kontaktieren der Stenose mittels des mit dem Inflationsmedium inflatierten Ballons, und
- Erzeugen von Strompulsen in der Spule mittels des Pulsgenerators zur Erzeugung eines zeitlich veränderlichen Magnetfeldes, so dass die Viskosität des Inflationsmediums im Balloninnenraum alternierend erhöht und abgesenkt wird, wobei durch das jeweilige Erhöhen der Viskosität Kraftstöße auf die Stenose ausgeübt werden, um diese aufzuweiten und/oder zu rekanalisieren.

Ein weiterer Aspekt der vorliegenden Erfindung betrifft die Verwendung einer ferromagnetischen Flüssigkeit zur Inflation eines Ballons eines Ballonkatheters.

Im Folgenden sollen Ausführungsformen der Erfindung sowie weitere Merkmale und Vorteile der Erfindung anhand der Figur erläutert werden. Es zeigt:
- Fig. 1: eine schematische Darstellung einer Ausführungsform eines erfindungsgemäßen Ballonkathetersystems, wobei die ferromagnetischen Partikel eines im Ballon befindlichen Ferrofluids nicht ausgerichtet sind, und
- Fig. 2: eine schematische Darstellung einer Ausführungsform eines erfindungsgemäßen Ballonkathetersystems mit mittels eines Magnetfeldes ausgerichteten ferromagnetischen Partikeln des Ferrofluids, wobei sich die Spule im proximalen Bereich des Ballonkatheters befindet.
- Fig. 3: eine schematische Darstellung einer Ausführungsform eines erfindungsgemäßen Ballonkathetersystems mit mittels eines Magnetfeldes ausgerichteten ferromagnetischen Partikeln des Ferrofluids, wobei sich die Spule im distalen Bereich des Ballonkatheters befindet.

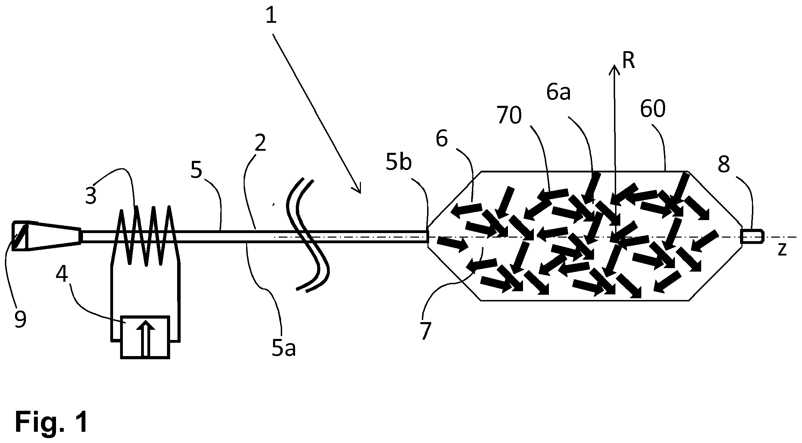

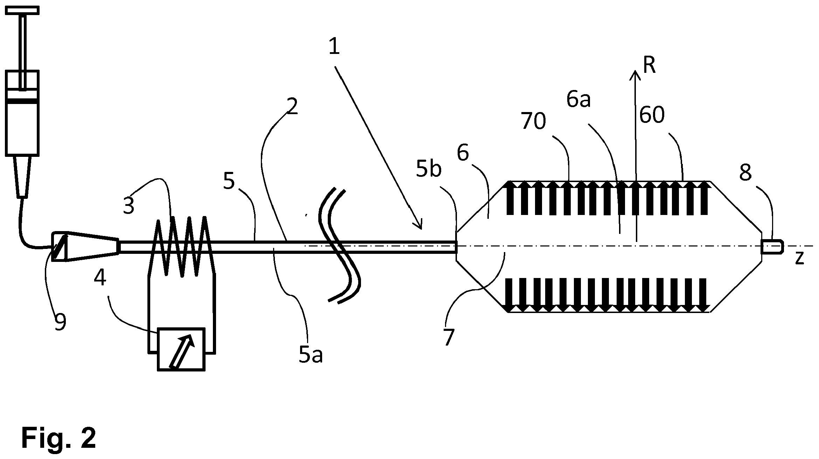

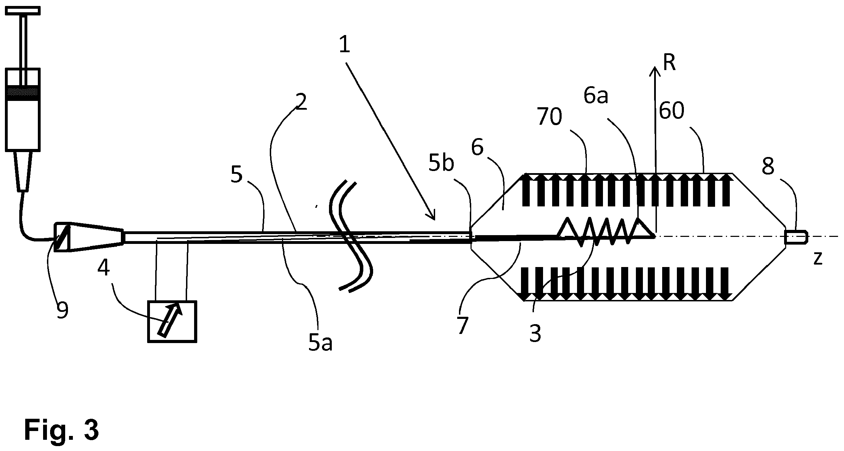

Die Erfindung betrifft, wie z.B. anhand der Ausführungsform gemäß der Fig. 1 ersichtlich ist, ein Ballonkathetersystem 1, das einen Ballonkatheter 2 mit einem in einer vorzugsweise axialen Richtung z erstreckten Ballon 6 aufweist, der einen Balloninnenraum 6a umgibt, der zur Expansion des Ballons 6 bzw. zum Inflatieren des Ballons 6 in einer vorzugsweise radialen Richtung R des Ballons 6 mit einem fluiden Inflationsmedium 7 befüllbar ist. Die jeweilige radiale Richtung R steht senkrecht auf der axialen Richtung z und weist nach außen zur Ballonwandung 60 des Ballons 6 hin. Weiterhin weist das Ballonkathetersystem 1 einen vorzugsweise in der axialen Richtung z erstreckten Katheterschaft 5 auf, wobei der Ballon 6 mit einem distalen Ende 5b des Katheterschafts 5 verbunden ist, und wobei der Katheterschaft 5 ein mit dem Balloninnenraum 6a verbundenes Lumen 5a umgibt, über das das Inflationsmedium 7 in den Balloninnenraum 6a einleitbar ist (z.B. mittels einer geeigneten Pumpe oder über eine Spritze wie in den Figuren 2 und 3 gezeigt). Der Ballon 6 ist vorzugsweise zylindersymmetrisch bezüglich der axialen Richtung z ausgebildet. Weiterhin kann der Ballonkatheter 2 an einem proximalen Ende einen Konnektor 9 (z.B. in Form eines Luer-Konnektors 9) aufweisen, der eine Öffnung zum Einleiten des Inflationsmediums 7 in das Lumen 5a bildet (bzw. zum Abziehen des Inflationsmediums 7 aus dem Balloninnenraum 6a bzw. Lumen 5a). Weiterhin kann der Ballonkatheter 2 ein nicht gezeigtes Führungsdrahtlumen zum Aufnehmen eines Führungsdrahtes aufweisen, der an einer distalen Katheterspitze 8 aus dem Ballonkatheter 2 herausführbar ist. Mittels des Führungsdrahtes kann der Ballon 6 des Ballonkatheters 2 zur Stenose geführt werden.

Erfindungsgemäß ist vorgesehen, dass das fluide Inflationsmedium 7 ein Ferrofluid ist, wobei das Ballonkathetersystem 1 weiterhin eine Spule 3 zum Erzeugen eines magnetischen Feldes aufweist und einen mit der Spule 3 elektrisch leitend verbundenen Pulsgenerator 4, der dazu konfiguriert ist, in der Spule 3 aufeinander folgende Strompulse zu erzeugen, so dass die Spule 3 ein zeitlich veränderliches Magnetfeld erzeugt, um eine Viskosität des Inflationsmediums 7 betragsmäßig periodisch zu variieren, so dass der Ballon 6 entsprechende Kraftstöße auf die Stenose ausüben kann, um eine Aufweitung/Rekanalisation des Stenose zu unterstützen.

Der Anteil der ferromagnetischen Partikel (z.B. Eisenpartikel) im Ferrofluid 7 kann z.B. 10% bis 30% des Gewichts und z.B. 5% bis 15% des Volumens des Ferrofluids 7 ausmachen. Die Dichte des Ferrofluids 7 kann z.B. zwischen 1,0 g·cm⁻³ und 4 g·cm⁻³ liegen. Derartige magnetische Flüssigkeiten (Ferrofluide) bestehen insbesondere aus kolloidal in einer Trägerflüssigkeit suspendierten kleinen ferromagnetischen Partikeln, insbesondere Eisenpartikeln, deren Durchmesser z.B. im Bereich von 10 nm liegen kann.

Beim mittels der Spule 3 und des Pulsgenerators 4 erzeugten Magnetfeld überwiegt hinsichtlich des Ferrofluids 7 der magnetorheologische Effekt. D.h., dass Viskositätsänderungen auf einer vergleichsweise kurzen Zeitskala erfolgen (z.B. unter einer Millisekunde), da nur die Partikel 70 am Magnetfeld ausgerichtet werden müssen. Somit sind Kraftänderungen innerhalb weniger Millisekunden realisierbar.

Diesbezüglich zeigt Fig. 1 einen Zustand des Ballonkathetersystems 1, bei dem kein Magnetfeld auf die Partikel 70 des Ferrofluids 7 einwirkt. Diese sind entsprechend ungeordnet hinsichtlich ihrer Ausrichtung und es liegt eine niedrige Viskosität der Inflationsmediums 7 vor.

Demgegenüber zeigt Fig. 2 schematisch einen Zustand mit Partikeln 70, deren magnetische Momente an dem kurzzeitig mittels eines Strompulses in der Spule 3 erzeugten Magnetfeldes ausgerichtet sind. Diesbezüglich ist eine Anzahl von mehreren hundert Strompulsen in der Spule pro Sekunde möglich.

Eine weitere Ausführungsform ist in Fig. 3 dargestellt, in der sich zum Unterschied zu Fig. 2 die Spule 3 im Bereich des Ballons befindet und über eine Leitung mit dem Pulsgenerator 4 verbunden ist.

Aufgrund der Erfindung sind mit Vorteil Stenosen durch Ermüdung mittels der erzeugten Kraftstöße mit weniger Druckkraft zerstörbar bzw. aufweitbar/rekanalisierbar.

Das erfindungsgemäße Prinzip eignet sich insbesondere zur Behandlung von vergleichsweise harten Verengungen/Stenosen eines Blutgefäßes eines Patienten.

Im Ergebnis ermöglicht die Erfindung eine Reduktion des Ballonplatzrisikos bei der Ballondilatation (tieferer Druck) sowie ein geringeres Rupturrisiko hinsichtlich des behandelten Blutgefäßes. Weiterhin lassen sich bestehende bzw. herkömmliche Ballonkathetersysteme vergleichsweise kostengünstig mit den notwendigen Komponenten (Spule, Ferrofluid und Pulsgenerator) nachrüsten.

## Patentansprüche

1. Ballonkathetersystem (1), mit:
- einem Ballonkatheter (2) aufweisend einen Katheterschaft (5) und einen an einem distalen Ende des Katheterschafts angeordneten Ballon (6), der eine dehnbaren Ballonwandung (60) aufweist, die einen Balloninnenraum (6a) des Ballons (6) umgibt, und
- einem fluiden Inflationsmedium (7), das zum Inflatieren des Ballons (6) über ein Lumen (5a) des Katheterschafts (5) in den Balloninnenraum (6a) einfüllbar ist,
**dadurch gekennzeichnet,**
**dass** das fluide Inflationsmedium (7) ein Ferrofluid ist, wobei das Ballonkathetersystem (1) weiterhin eine Spule (3) zum Erzeugen eines magnetischen Feldes aufweist und einen mit der Spule (3) verbundenen Pulsgenerator (4), der dazu konfiguriert ist, in der Spule (3) aufeinander folgende Strompulse zu erzeugen, so dass die Spule (3) ein zeitlich veränderliches Magnetfeld erzeugt, um eine Viskosität des Inflationsmediums (7) alternierend zu erhöhen und zu senken, wenn sich das Inflationsmedium (7) im Balloninnenraum (6a) befindet.

2. Ballonkathetersystem nach Anspruch 1, **dadurch gekennzeichnet, dass** das Ferrofluid eine Wasserdispersion von Eisenoxidpartikeln ist, die vorzugsweise aus Magnetit (Fe₃O₄) und Maghemit (γ-Fe₂O₃) bestehen.

3. Ballonkathetersystem nach Anspruch 2, **dadurch gekennzeichnet, dass** das Inflationsmedium (7) einen Massenanteil an ferromagnetischen Partikeln (70) im Bereich von 10 Gew.-% bis 30 Gew.-% aufweist.

4. Ballonkathetersystem nach Anspruch 2 oder 3, **dadurch gekennzeichnet, dass** das Inflationsmedium (7) einen Volumenanteil an ferromagnetischen Partikeln (70) im Bereich von 5 Vol.-% bis 15 Vol.-% aufweist.

5. Ballonkathetersystem nach einem der vorhergehenden Ansprüche, **dadurch gekennzeichnet, dass** die Dichte des Inflationsmediums (7) im Bereich von 1,0 g·cm⁻³ bis 4 g·cm⁻³ liegt.

6. Ballonkathetersystem nach einem der Ansprüche 2 bis 5, **dadurch gekennzeichnet, dass** die ferromagnetischen Partikel (70) einen Durchmesser im Bereich von 5 nm bis 15 nm aufweisen, wobei der Durchmesser vorzugsweise 10 nm beträgt.

7. Ballonkathetersystem nach einem der vorhergehenden Ansprüche, **dadurch gekennzeichnet, dass** die Spule mobil ist.

8. Ballonkathetersystem nach einem der vorhergehenden Ansprüche, **dadurch gekennzeichnet, dass** der Pulsgenerator (4) dazu konfiguriert ist, Strompulse in der Spule (3) zu erzeugen, die eine Wiederholrate im Bereich von 50 bis 150 Hz aufweisen.

9. Verfahren zum Aufweiten und/oder Rekanalisieren einer Stenose mittels eines Ballonkathetersystems (1) nach einem der vorhergehenden Ansprüche, aufweisend die Schritte:
- Kontaktieren der Stenose mittels des mit dem Inflationsmedium (7) inflatierten Ballons (6), und
- Erzeugen von Strompulsen in der Spule (3) mittels des Pulsgenerators (4) zur Erzeugung eines zeitlich veränderlichen Magnetfeldes, so dass die Viskosität des Inflationsmediums (7) im Balloninnenraum (6a) alternierend erhöht und abgesenkt wird, wobei durch das jeweilige Erhöhen der Viskosität Kraftstöße auf die Stenose ausgeübt werden, um eine Aufweitung und/oder Rekanalisierung der Stenose zu unterstützen.

10. Verwendung eines Ferrofluids zur Inflation eines Ballons eines Ballonkatheters.
